(19) Europäisches Patentamt
European Patent Office
Office européen des brevets

(11) **EP 4 197 985 A1**

(12) **EUROPÄISCHE PATENTANMELDUNG**

(43) Veröffentlichungstag:
**21.06.2023  Patentblatt 2023/25**

(21) Anmeldenummer: **21215599.8**

(22) Anmeldetag: **17.12.2021**

(51) Internationale Patentklassifikation (IPC):
*C04B 28/02* (2006.01)  *C04B 40/00* (2006.01)
*C04B 40/06* (2006.01)  *G01N 33/38* (2006.01)

(52) Gemeinsame Patentklassifikation (CPC):
(C-Sets verfügbar)
**G01N 33/383; C04B 28/02; C04B 40/0032;
C04B 40/0625** (Forts.)

(84) Benannte Vertragsstaaten:
**AL AT BE BG CH CY CZ DE DK EE ES FI FR GB
GR HR HU IE IS IT LI LT LU LV MC MK MT NL NO
PL PT RO RS SE SI SK SM TR**
Benannte Erstreckungsstaaten:
**BA ME**
Benannte Validierungsstaaten:
**KH MA MD TN**

(71) Anmelder: **HeidelbergCement AG
69120 Heidelberg (DE)**

(72) Erfinder:
• **Pfeuffer, Markus
69120 Heidelberg (DE)**
• **Zwolinski, Marek
69120 Heidelberg (DE)**

(74) Vertreter: **Zellentin & Partner mbB Patentanwälte
Rubensstraße 30
67061 Ludwigshafen (DE)**

(54) **VERFAHREN ZUR NUTZUNG VON RESTWASSER**

(57)   Verfahren zur Herstellung von hydraulischen Baumaterialien, bei dem als Zugabewasser zumindest teilweise Restwasser verwendet wird, wobei die Dichte des einströmenden Restwassers kontinuierlich gemessen wird und bei Überschreitung eines gewählten Grenzwertes der Dichte so viel Frischwasser oder Restwasser geringerer Dichte zugeführt wird, dass die Dichte unterhalb des Grenzwertes liegt, wobei eine Menge von mindestens einer festen Komponente des hydraulischen Baumaterials in Abhängigkeit von einer aus der Dichte berechneten Gesamtfeststoffmenge im Restwasser reduziert wird.

EP 4 197 985 A1

(52) Gemeinsame Patentklassifikation (CPC): (Forts.)

C-Sets
**C04B 28/02, C04B 14/06, C04B 14/28,
C04B 18/08, C04B 18/141, C04B 18/146,
C04B 20/008, C04B 22/0033, C04B 22/0046,
C04B 40/0032, C04B 40/0625, C04B 2103/32;
C04B 40/0032, C04B 22/0033, C04B 22/0046,
C04B 40/0625;
C04B 40/0625, C04B 14/06, C04B 14/28,
C04B 18/08, C04B 18/141, C04B 18/146,
C04B 20/008, C04B 22/0033, C04B 22/0046,
C04B 28/02, C04B 40/0032, C04B 2103/32**

**Beschreibung**

Die vorliegende Erfindung betrifft ein Verfahren zur Herstellung von hydraulischen Baumaterialien bei dem Restwasser zumindest teilweise als Zugabewasser verwendet wird.

Stand der Technik

Restwasser fällt an vielen Stellen im Prozess der Betonherstellung und Verarbeitung an, beispielsweise bei der Aufbereitung von Restbeton oder -mörtel, der Reinigung der Fahrmischertrommel und anderer Mischer, der Reinigung von Betonpumpen, beim Sägen, Schleifen und Wasserstrahlen von Beton, beim Waschen der Gesteinskörnung und während der Herstellung des Frischbetons. Restwasser enthält in schwankenden Konzentrationen Fein- und Feinstteile des ausgewaschenen Restbetons oder -mörtels, deren Korngröße in der Regel unter 0,25 mm liegt.

Restwasser darf gemäß der Norm DIN EN 1008 als Zugabewasser verwendet, bzw. frischem Zugabewasser beigemengt werden. Die Feinanteile im Restwasser müssen bei der Mischungsberechnung berücksichtigt werden. Für Betone mit besonderen Anforderungen, wie z. B. hochfeste Betone oder Luftporenbetone, darf kein Restwasser verwendet werden. Bei der Herstellung von Sichtbetonbauteilen wird empfohlen, auf Restwasser zu verzichten. Folgende Anforderungen werden an Restwasser zur Verwendung in der Betonherstellung gestellt:

- Feststoffe im Restwasser müssen homogen verteilt sein oder in einem Absetzbecken abgeschieden werden.
- Die Dichte des Restwassers ist mindestens einmal täglich zum Zeitpunkt der zu erwartenden höchsten Dichte zu ermitteln. Günstiger ist eine kontinuierliche Dichtebestimmung über den Tag.
- Der Feststoffgehalt ist aus der Dichtebestimmung und der zugegebenen Restwassermenge nach einer Tabelle zu ermitteln und bei der Betonzusammensetzung zu berücksichtigen.
- Erfüllt das Restwasser die Anforderungen von DIN EN 1008 nicht, darf so viel davon zugegeben werden, dass die Grenzwerte der Anforderungen bezogen auf das gesamte Zugabewasser eingehalten werden.

Aus DE 197 47 283 A1 ist es bekannt, bei einem Verfahren zur chargenweisen Herstellung von Betonmischungen, bei dem als Zugabewasser Restwasser verwendet wird, das über einen mit einer Wasserwaage gekoppelten Wasservorratsbehälter dosiert wird. Aus dem Volumenstrom des Restwassers einerseits und der Gewichtskraft des in den Wasservorratsbehälter eingeströmten Restwassers andererseits wird die Dichte des Restwassers ermittelt und auf der Grundlage dieser Dichte die der Rezeptur der Betonmischung entsprechende zusätzlich erlaubte Menge an Restwasser bestimmt und nachdosiert. Bevorzugt erfolgt die Dichtebestimmung in einem vorgegebenen Intervall zwischen Beginn und Ende der Zugabewasserdosierung. Die Ermittlung der zu dosierenden Restwassermenge soll über eine automatische Auswerteinrichtung berechnet oder anhand von Tabellen bestimmt werden. Mit diesem Verfahren wird eine normgemäße Wiederverwendung von Restwasser ermöglicht. Allerdings kann Restwasser als Zugabewasser die Betoneigenschaften auch dann beeinflussen, wenn die Anforderungen der DIN EN 1008 eingehalten wird.

DE 20 2019 005 624 U1 und WO 2020/030729 A1 schlagen ein System und Verfahren vor, mit dem Rück- und Restbeton aus der Reinigung von Betonmischanlagen und Fahrmischern vom Restwasser getrennt und nutzbar gemacht werden soll. Das aufbereitete Restwasser kann in der Reinigung wiederverwendet werden, um ein geschlossenes System zu schaffen. Eine Nutzung als Zugabewasser ist ebenfalls erwähnt. Zur Kontrolle der Aufbereitung, d.h. der Abscheidung der Feststoffe, wird u.a. eine Dichtemessung vorgeschlagen. Die vorgeschlagene Abtrennung von Feststoffen ist aufwändig. Das Problem einer möglichen Beeinflussung der Betoneigenschaften durch den Einsatz von Restwasser als Zugabewasser wird nicht angesprochen oder gelöst.

Es besteht daher weiter die Aufgabe, ein Verfahren zur Restwassernutzung ohne nachteilige Auswirkungen auf den Beton zu bereitzustellen.

Zusammenfassung der Erfindung

Überraschend wurde nun gefunden, dass eine kontinuierliche Dichtemessung in Kombination mit einer Anpassung nicht nur der Frischwassermenge sondern auch der Mengen an festen Komponenten in der Betonrezeptur in der Lage ist, gleichbleibende und gute Betoneigenschaften zu gewährleisten. Außerdem lassen sich die Kosten senken, indem feste Komponenten eingespart werden. Eine aufwändige Abtrennung von Feststoffen wird vermieden.

Die oben genannte Aufgabe wird daher durch ein Verfahren zur Herstellung von hydraulischen Baumaterialien gelöst, bei dem als Zugabewasser zumindest teilweise Restwasser verwendet wird, wobei die Dichte des einströmenden Restwassers kontinuierlich gemessen wird und bei Überschreitung eines gewählten Grenzwertes der Dichte so viel Frischwasser oder Restwasser geringerer Dichte zugeführt wird, dass die Dichte unterhalb des Grenzwertes liegt, und wobei eine Menge von mindestens einer festen Komponente des hydraulischen Baumaterials, insbesondere von Zement, Zusatzstoff(en) und/oder Sand, in Abhängigkeit von einer aus der Dichte berechneten Gesamtfeststoffmenge im Restwasser reduziert wird.

Das erfindungsgemäße Verfahren erlaubt die Herstellung von Beton mit gleichbleibenden Eigenschaften bezüglich Konsistenz, Festigkeit etc. Gleichzeitig wird Restwasser mit minimalem Aufwand wiederverwendet. Zudem werden Zement, Zusatzstoff(e) und Sand eingespart, da deren Gehalt im Restwasser auf Basis der kontinuierlichen Dichtemessung für die Rezeptur berücksichtigt und entsprechend weniger frischer Zement, Zusatzstoff(e) und/oder Sand eingesetzt wird. Das spart Kosten und ist vorteilhaft für die Umwelt, weil Ressourcen gespart werden.

Definitionen

Restwasser bezeichnet Wasser, welches im Prozess der Betonherstellung und -verarbeitung anfällt, insbesondere:

- Abwasser aus der Aufbereitung von Restbeton oder -mörtel,
- Abwasser aus der Reinigung der Fahrmischertrommel und anderer Mischer, der Reinigung von Betonpumpen und anderer Vorrichtungen zum Verarbeiten von Beton einschließlich Handgeräten
- Wasser vom Sägen, Schleifen und Wasserstrahlen von Beton,
- Abwasser vom Waschen der Gesteinskörnung und
- Abwasser aus der Herstellung des Frischbetons.

Hydraulisches Baumaterial meint im Rahmen der vorliegenden Erfindung ein zunächst plastisches, in der Regel fließfähiges, und dann hydraulisch erhärtendes Material, welches Zement und Wasser sowie meist auch Gesteinskörnung und ggfs. Zusatzstoffe und/oder Zusatzmittel enthält. Beton und Mörtel sind typische hydraulische Baumaterialien, aber auch Fließestrich, Fugenmasse, Fliesenkleber, etc. zählen dazu, soweit die Erhärtung durch eine hydraulische Reaktion von Zement erfolgt.

Mit Zement wird sowohl ein mit oder ohne Zusatz weiterer Komponenten gemahlener Klinker bezeichnet, als auch andere hydraulisch erhärtende Materialien und Mischungen, beispielsweise aber nicht ausschließlich Sulfathüttenzement, Geopolymerzement und durch hydrothermale Umsetzung erhaltener Belitzement. Im Rahmen der vorliegenden Erfindung meint Klinker ein Sinterprodukt, welches durch Brennen eines Ausgangsmaterials bei erhöhter Temperatur erhalten wird und zumindest eine hydraulisch reaktive Phase enthält. Brennen meint die Aktivierung durch Veränderungen einer oder mehrerer der Eigenschaften Chemismus, Kristallinität, Phasenzusammensetzung, dreidimensionale Anordnung und Bindungsverhalten der Gerüstatome durch die Zufuhr von thermischer Energie. Das Ausgangsmaterial kann im Einzelfall auch ein einzelner Rohstoff sein, wenn dieser alle gewünschten Substanzen in der richtigen Relation enthält, das ist aber die Ausnahme. Das Ausgangsmaterial kann auch Mineralisatoren enthalten. Als Mineralisatoren werden Stoffe bezeichnet, welche als Flussmittel wirken und/oder die Temperatur senken, die zur Bildung einer Schmelze notwendig ist, und /oder solche, die die Bildung der Klinkerverbindung fördern, wie zum Beispiel durch Mischkristallbildung und/oder Phasenstabilisierung. Mineralisatoren können im Ausgangsmaterial als Bestandteil enthalten sein oder gezielt zugefügt werden.

Gesteinskörnung bezeichnet natürliche und künstliche Gesteinskörner. Gesteinskörnung wird anhand der Korngröße in Kies und Sand unterteilt, wobei Kies typischerweise Körner von 4 mm bis 32 mm umfasst und Sand Körner mit maximalen Korngrößen von 4 mm. Die Benennung erfolgt nach der Größe des Kleinst- und des Größtkorns, welche durch genormte Siebvorgänge ermittelt werden. Gesteinskörnung macht normalerweise den mengenmäßig größten Anteil fester Komponenten von hydraulischen Baumaterialien aus.

Zusatzmittel werden dem hydraulischen Baumaterial beigefügt, um die Eigenschaften im frischen Zustand wie auch im erhärteten Zustand zu optimieren. Überwiegend handelt es sich um organische Verbindungen, die in sehr geringen Mengen zugegeben werden und wirksam sind. Dazu zählen beispielsweise, aber nicht ausschließlich, Fließmittel, Betonverflüssiger, und Luftporenbildner.

Zusatzstoffe sind weitere Materialien, die dem Zement oder dem hydraulischen Baumaterial beigefügt werden, vor allem um die Eigenschaften des erhärteten Materials zu verändern. In der Regel werden größere Mengen als bei den Zusatzmitteln eingesetzt. Zu den Zusatzstoffen zählen solche vom Typ 1, vor allem Füllstoffe, Fasern, Pigmente und Polymere, die bezüglich der hydraulischen Reaktion als inert angesehen werden. Allerdings liefern bestimmte Zusatzstoffe wie z.B. Kalksteinmehl oder Mikrosilica zumindest in geringen Mengen Ionen, die an der hydraulischen Reaktion teilnehmen und insofern nicht inert sind. Als Zusatzstoffe vom Typ 2, auch Klinkerersatzmaterialien genannt, werden puzzolanische und/oder latent-hydraulische Materialien bezeichnet, die in einem Zement bzw. hydraulischen Baumaterial einen Teil des Klinkers ersetzen. Latent-hydraulische Materialien weisen eine Zusammensetzung auf, die in Kontakt mit Wasser eine hydraulische Erhärtung ermöglicht, wobei typischerweise für eine Erhärtung in technisch nutzbaren Zeiträumen ein Anreger nötig ist. Mit Anreger oder Aktivator ist ein Material gemeint, welches die Erhärtung von latent-hydraulischen Materialien beschleunigt. Es kann sich um einen Zusatz handeln, etwa Sulfat, Calciumoxid oder Calciumhydroxid und/oder um Produkte der hydraulischen Reaktion des Klinkers, z.B. setzen Calciumsilkate bei das Erhärtung Calciumhydroxid frei, welches als Anreger wirkt. Puzzolanische Materialien sind durch einen Gehalt an reaktionsfähigem Siliziumdioxid und/oder Aluminiumoxid gekennzeichnet, welche bei der Hydratation eines Bindemittels mit in der wässrigen Phase vorhandenen Calciumionen zu Festigkeits-bildenden Calciumsilikat- bzw. Aluminathydratphasen umgesetzt werden. In der Praxis ist die Grenze zwischen latent-hydraulischen und puzzolanischen Materialien fließend, z.B. können Flugaschen je nach Gehalt an Calciumoxid puzzolanisch oder latent-hydraulische Materialien sein. Mit Klinkerersatzmaterial sind sowohl latent-hydraulische als auch puzzolanische Materialien gemeint.

Detaillierte Beschreibung der Erfindung

Erfindungsgemäß erfolgt für die Verwendung von Restwasser als (Teil des) Zugabewasser(s) für die Herstellung von hydraulischem Baumaterial eine kontinuierliche Dichtemessung des Restwassers. Auf Basis der gemessenen Dichte wird der Anteil Restwasser am Zugabewasser geregelt. Dabei erfolgt die Messung nicht nur einmal am Tag sondern laufend. So lassen sich falsche Annahmen zum Gehalt an Feststoffen im Zugabewasser sicher vermeiden. Die Kontrolle der Dichte und Zugabe von Frischwasser oder Restwasser niedrigerer Dichte inklusive der Bestimmung der notwendigen Menge erfolgt in der Regel automatisch. Geeignete Regelungen sind an sich bekannt und kommerziell erhältlich.

Vorteilhaft wird mit derselben Regelung auch die Zugabe einer oder mehrerer fester Komponenten in der Rezeptur vermindert. Welche Komponente bis zu welcher Menge verringert wird, hängt vom Restwasser und der Rezeptur ab und wird dementsprechend festgelegt.

Die Rezeptur, d.h. die jeweiligen Zielmengen der Komponenten, hängt vor allem vom Verwendungszweck des Baumaterials ab, aber auch von der zur Verfügung stehenden bzw. gewählten Qualität der Komponenten. Beispielsweise erreicht feinerer Zement eine höhere Festigkeit, somit ist bei einer bestimmten benötigten Festigkeit für einen Beton weniger feiner Zement im Vergleich zu Zement mit normaler Feinheit nötig.

Typische Betonrezepturen umfassen folgende Komponenten:

- 110 bis 600 kg/m$^3$, vorzugsweise 200 bis 400 kg/m$^3$, besonders bevorzugt 280 bis 350 kg/m$^3$ Zement
- 300 bis 3500 kg/m$^3$, vorzugsweise 1200 bis 2400 kg/m$^3$, besonders bevorzugt 1.900 bis 1.800 kg/m$^3$ Gesteinskörnung
- 110 bis 220 kg/m$^3$, vorzugsweise 150 bis 200 kg/m$^3$, besonders bevorzugt 177 bis 185 kg/m$^3$ Wasser
- 0 bis 600 kg/m$^3$, vorzugsweise 1 bis 100 kg/m$^3$, besonders bevorzugt 5 bis 40 kg/m$^3$ Zusatzstoffe (z.B. Flugasche, Kalksteinmehl, ...)
- 0 bis 1,5 Gew.-%, vorzugsweise 0,01 bis 1,0 Gew.-%, besonders bevorzugt 0,1 bis 0,6 Gew.-% vom Zementgewicht Zusatzmittel (z.B. Fließmittel).

Wasser bezieht sich hier auf die reine Wassermenge, d.h. die Menge Restwasser ist höher und wird so festgelegt, dass die gewünschte Menge reines Wasser im Zugabewasser enthalten ist. Es wird also auf Basis der kontinuierlich gemessenen Dichte berechnet, wieviel Wasser im Restwasser enthalten ist und daraus die notwendige Zugabemenge Restwasser. Analog wird für die feste(n) Komponente(n) verfahren, die aufgrund der im Restwasser enthaltenen Anteile in ihrer Menge reduziert werden.

Normalerweise wird ein Wasser/Zementwert im Bereich von 0,40 bis 0,60 eingestellt. In die Zementmenge werden dabei neben dem Zement auch die Zusatzstoffe vom Typ 2 (Klinkerersatzmaterialien) einberechnet. Zusatzstoffe vom Typ 1, wie Pigmente oder Fasern, werden ebenso wie die Gesteinskörnung nicht berücksichtigt.

Zur Herstellung des hydraulischen Baumaterials werden alle Komponenten der gewählten Rezeptur einem Mischer zugeführt. Das kann sowohl ein stationärer Mischer, z.B. in einem Fertigteilwerk oder auf einer Baustelle, als auch ein Fahrmischer sein. Der Einsatz von Restwasser für die Herstellung von Ortbeton erfordert die Verfügbarkeit eines Sammelbehälters für Restwasser sowie der Dichtemesseinrichtung und Regelung auf der Baustelle.

In der Regel erfolgt die Dosierung durch Einwaage der Komponenten. Bei Zusatzmitteln ist auch eine volumetrische Dosierung üblich, da diese häufig als Lösung oder Suspension vorliegen. In jedem Fall muss eine Dichtemesseinrichtung kontinuierlich die Dichte des Restwassers bestimmen. Die Dosierung muss eine dementsprechende Zugabe von Frischwasser bzw. Restwasser niedrigerer Dichte ermöglichen sowie die Regelung der Zugabemengen von mindestens einer festen Komponente, bevorzugt der Komponenten Zement und Sand, am meisten bevorzugt der Komponenten Zement, Zusatzstoffe und Sand.

Als Dichtemesseinrichtung für das Restwasser wird vorzugsweise ein optischer Sensor verwendet, der die Trübung des Restwassers bestimmt, insbesondere mit diffusem Infrarotlicht. Bei dieser Art der Dichtemessung haben Kratzer oder Verunreinigungen an der Messoptik der Messeinrichtung keinen oder zumindest einen sehr geringen Einfluss auf die Messung. Das ist wichtig, weil Restwasser erhebliche Mengen an Feststoffen enthält. Bei kontinuierlicher Messung weist die Messoptik so innerhalb kurzer Zeit, z.B. weniger Tage, Kratzer auf, Verunreinigungen können bereits nach wenigen Minuten auftreten. Ein weiterer Vorteil der optischen Messung ist die Möglichkeit, nur die Messoptik im Restwasser anzuordnen und die Signale über einen Lichtwellenleiter an eine entfernt angeordnete Auswerteelektronik zu senden. Brauchbare Messeinrichtungen sind z.B. von Chemtronic Waltemode GmbH, DE, Werne & Thiel sensortechnik GbR, DE, oder Exner Process Equipment GmbH, DE, erhältlich. Zu diesen Messeinrichtungen sind auch Regelungen verfügbar.

Zusätzlich werden Informationen zu den im Restwasser befindlichen Feststoffen benötigt. Diese können entweder - z.B. bei fest installierten Anlagen - aus Kenntnissen über den Ursprung des Restwassers stammen oder sie werden in geeigneten Abständen ermittelt. Die Rohdichte und die Sieblinie des im Restwasser enthaltenen Feststoffes kann z.B. 1 Mal pro Halbjahr ermittelt werden. Zur Ermittlung der Sieblinie wird der Feststoff getrocknet und per Siebanalyse die Sieblinie bestimmt. Die Rohdichte wird mit dem Pyknometer ermittelt.

Über diese Informationen wird aus der kontinuierlich gemessenen Dichte eine Zusammensetzung des Restwassers bestimmt, auf deren Basis sowohl die ggfs. nötige Zugabe von Frischwasser oder Restwasser geringerer Dichte, als auch die konkrete Reduzierung der Menge an fester Betonkomponente bzw. Mengen fester Betonkomponenten fortlaufend berechnet wird. Vorzugsweise erfolgt die Zugabe von Frischwasser oder Restwasser geringerer Dichte sowie Anpassung der Menge(n) fester Betonkomponente(n) automatisch durch die

Regelung. Die Feststoffmenge im Restwasser ermittelt sich nach der Formel:

$$m_{Feststoff} = m_{Restwasser} \cdot ((1 - \rho_{Restwasser)} * \rho_{Feststoff}) / (1 - \rho_{Feststoff}) \cdot \rho_{Restwasser}))$$

mit

$m_{Feststoff}$ = Masse Feststoff im Restwasser in kg/l
$m_{Restwasser}$ = Masse Restwasser in kg/l
$\rho_{Restwasser}$ = Dichte Restwasser in kg/l
$\rho_{Feststoff}$ = Dichte Feststoff in kg/l.

Die festen Betonkomponenten werden im allgemeinen um die gesamte Menge der zugeführten Feststoffe aus dem Restwasser reduziert. Dabei wird vorzugsweise nach der Reihenfolge Zement, Zusatzstoffe Typ 2, Zusatzstoffe Typ 1, Sand reduziert, solange wie noch Teile der ermittelten Feststoffmenge aus dem Restwasser zur Verfügung stehen. Die detaillierten Festlegung welche Betonkomponente in welcher Menge reduziert werden können, wird durch das betreuende Betonlabor anhand folgender Kriterien festgelegt:

- Zement: pro 1,0 N/mm$^2$ an überschüssiger Betondruckfestigkeit über der Mindestanforderung werden bis zu 10 kg/m$^3$ an Zement ersetzt. Die überschüssige Betondruckfestigkeit über der Mindestanforderung ergibt sich aus der gemäß DIN EN 206-1 tatsächlich gemessenen Betondruckfestigkeit im Alter von 28 Tagen abzüglich der für die jeweilige Betonrezeptur festgelegten charakteristischen Solldruckfestigkeit. Ein werkspezifische definiertes Vorhaltemaß wird vorzugsweise zusätzlich abgezogen.
- Zusatzstoffe: Zusatzstoffe vom Typ 1, wie Kalksteinmehl, können bis zu maximal der nach Rezeptur enthaltenen Menge ausgetauscht werden. Bei Zusatzstoffen vom Typ 2, d.h. puzzolanischen oder latent hydraulischen Zusatzstoffen, werden pro 1,0 N/mm$^2$ an überschüssiger Betondruckfestigkeit nach 28 Tagen (bestimmt wie zuvor) bis zu 25 kg/m$^3$ ersetzt.
- Sand: wird bis zur maximal in der Rezeptur eingesetzten Menge ersetzt.

In einer ersten Ausführungsform wird in einem Transportbetonwerk für die Verwendung von Restwasser, welches bei der Reinigung der Fahrmischer anfällt, als Zugabewasser für den Transportbeton die Dichte des Restwassers im Zulauf zum Zwangsmischer bestimmt. Die bei einer gemessenen Dichte im Restwasser enthaltenen Mengen der festen Betonkomponenten werden einmalig ermittelt und in der Regelung hinterlegt. So kann aus der gemessenen Dichte direkt eine evtl. nötige Zugabe Frischwasser oder Restwasser geringerer Dichte ermittelt und veranlasst werden. Außerdem bestimmt die Regelung aus der Dichte die anzuwendende Reduktion an festen Betonkomponenten.

In einer zweiten Ausführungsform wird Restwasser in einem Sammelbecken gespeichert und in Abständen von einigen Stunden, z.B. 1, 2, 5, 6, 10, oder 12 Stunden, bis hin zu wenigen Tagen, z.B. 1, 2 oder 3 Tagen, die Art und Verteilung der enthaltenen Feststoffe gemessen. Die Messhäufigkeit richtet sich danach, wie variabel das dem Sammelbecken zugeführte Restwasser zusammengesetzt ist. Diese Information wird dann zusammen mit den kontinuierlich gemessenen Dichtewerten der Regelung zugeführt, die daraus eine evtl. nötige Zugabe Frischwasser oder Restwasser geringerer Dichte und anzuwendende Reduktion an festen Betonkomponenten bestimmt und vorzugsweise auch veranlasst.

In einer dritten Ausführungsform wird in einem Transportbetonwerk für die Verwendung von Restwasser aus einem Sammelbecken als Zugabewasser für den Transportbeton die Dichte des Restwassers dauerhaft im Zulauf zum Zwangsmischer bestimmt. Bei jeder Produktionscharge steuert die Regelung automatisiert die anhand der gemessenen Dichte nötige Zugabe von Frischwasser und Restwasser. Außerdem bestimmt die Regelung aus der Dichte die anzuwendende Reduktion an festen Betonkomponenten und zieht diese automatisch pro Charge ab.

Die Erfindung soll anhand der folgenden Beispiele erläutert werden, ohne jedoch auf die speziell beschriebenen Ausführungsformen beschränkt zu sein. Soweit nichts anderes angegeben ist oder sich aus dem Zusammenhang zwingend anders ergibt, beziehen sich Prozentangaben auf das Gewicht, im Zweifel auf das Gesamtgewicht der Mischung.

Die Erfindung bezieht sich auch auf sämtliche Kombinationen von bevorzugten Ausgestaltungen, soweit diese sich nicht gegenseitig ausschließen. Die Angaben "etwa" oder "ca." in Verbindung mit einer Zahlenangabe bedeuten, dass zumindest um 10 % höhere oder niedrigere Werte oder um 5 % höhere oder niedrigere Werte und in jedem Fall um 1 % höhere oder niedrigere Werte eingeschlossen sind.

Beispiel 1

In einem Transportbetonwerk wird das beim Reinigen der Fahrmischer anfallende Restwasser in einem Becken gesammelt. Durch Messungen wurde festgestellt, dass bei einer Dichte von 1,07 kg/l ca. 0,134 kg/l Feststoffe in dem Restwasser enthalten sind. Das entspricht bei einem typischen Ansatz mit 180 kg/m$^3$ Zugabewasser ca. 24 kg/m$^3$ festen Betonkomponenten. Für eine Betonrezeptur mit 295 kg Zement, 1.757 kg Gesteinskörnung, davon 583 kg Sand, 50 kg Flugasche und 2,4 kg Fließmittel wurde festgelegt, dass Zement in einer Menge von ca. 21 % und Sand 0/2 in einer Menge von 79 % der über die Dichte bestimmten Feststoffmenge reduziert wird. Mit einem optischen Sensor wurde in der jeweils produzierten Charge die Restwasserdichte am Wasserzulauf zur Wasserwaage gemessen. Dieser Wert wurde in der darauffolgenden Charge zur Ermittlung des Anteils von Frisch- und Restwasser und der Ausgangsstoffreduzierung genutzt. Lag beispielsweise die Dichte bei 1,09 kg/l so wurde durch die Regelung für eine benötigte Zugabewassermenge von 180 kg/m$^3$ eine Verwendung von 154 kg/m$^3$ Restwasser und 25 kg/m$^3$ Frischwasser eingestellt: 1,09 kg/l (25 kg/m$^3$ Frischwasser $\times$ 1,00 kg/l + 154 kg/m$^3$ Restwasser $\times$ 1,09 kg/l)/180 kg/m$^3$ Gesamtwasser = 1,07 kg/l Dichte im Zugabewasser. Außerdem wurden die Bindemittelmenge um 5 kg/m$^3$ und der Sand 0/2 um 19 kg/m$^3$ reduziert. Die laufende Qualitätsüberwachung des Transportbetons bestätigte gleichbleibende und den Anforderungen entsprechende Eigenschaften.

**Patentansprüche**

1.   Verfahren zur Herstellung von hydraulischen Baumaterialien enthaltend Zement und Zugabewasser, bei dem als Zugabewasser zumindest teilweise Restwasser verwendet wird, wobei die Dichte des einströmenden Restwassers kontinuierlich gemessen wird und bei Überschreitung eines gewählten Grenzwertes der Dichte so viel Frischwasser oder Restwasser geringerer Dichte zugeführt wird, dass die Dichte unterhalb des Grenzwertes liegt,
   **dadurch gekennzeichnet, dass** eine Menge von mindestens einer festen Komponente des hydraulischen Baumaterials in Abhängigkeit von einer aus der Dichte gemäß der Formel:

$$m_{Feststoff} = m_{Restwasser} \bullet ((1 - \rho_{Restwasser)} * \rho_{Feststoff}) / (1 - \rho_{Feststoff}) \bullet \rho_{Restwasser}))$$

   mit

   $m_{Feststoff}$ = Masse Feststoff im Restwasser in kg/l,
   $m_{Restwasser}$ = Masse Restwasser in kg/l,
   $\rho_{Restwasser}$ = Dichte Restwasser in kg/l,
   $\rho_{Feststoff}$ = Dichte Feststoff in kg/l,
   berechneten Gesamtfeststoffmenge im Restwasser reduziert wird.

2.   Verfahren gemäß Anspruch 1, **dadurch gekennzeichnet, dass** eine Menge an Zement reduziert wird, insbesondere bis zu 10 kg/m$^3$ Zement pro 1,0 N/mm$^2$ gemäß DIN EN 206-1 nach 28 Tagen gemessener Betondruckfestigkeit, welche die der für das Baumaterial festgelegten Festigkeit nach 28 Tagen übersteigt, ersetzt werden.

3.   Verfahren gemäß Anspruch 1 oder 2, **dadurch gekennzeichnet, dass** das Baumaterial Zusatzstoffe vom Typ 2 enthält und eine Menge an Zusatzstoff(en) vom Typ 2 reduziert wird, insbesondere bis zu 25 kg/m$^3$ Zusatzstoffe pro 1,0 N/mm$^2$ gemäß DIN EN 206-1 nach 28 Tagen gemessener Betondruckfestigkeit, welche die der für das Baumaterial festgelegten Festigkeit nach 28 Tagen übersteigt, ersetzt werden.

4.   Verfahren gemäß einem der Ansprüche 1 bis 3, **dadurch gekennzeichnet, dass** das Baumaterial Zusatzstoffe vom Typ 1 und/oder Sand enthält und eine Menge an Zusatzstoff vom Typ 1 und/oder Sand reduziert wird, insbesondere bis zur maximal nach Rezeptur enthaltenen Menge.

5. Verfahren gemäß Anspruch 4, **dadurch gekennzeichnet, dass** das Baumaterial Zusatzstoffe vom Typ 1 und/oder 2 und Sand enthält und die festen Komponenten in der Reihenfolge Zement, Zusatzstoff(e), Sand ersetzt werden.

6. Verfahren gemäß einem der Ansprüche 1 bis 5, **dadurch gekennzeichnet, dass** das hydraulische Baumaterial ein Beton mit folgenden Komponenten ist:

- 110 bis 600 kg/m$^3$, vorzugsweise 200 bis 400 kg/m$^3$, besonders bevorzugt 280 bis 350 kg/m$^3$ Zement
- 300 bis 3500 kg/m$^3$, vorzugsweise 1200 bis 2400 kg/m$^3$, besonders bevorzugt 1.900 bis 1.800 kg/m$^3$ Gesteinskörnung
- 110 bis 220 kg/m$^3$, vorzugsweise 150 bis 200 kg/m$^3$, besonders bevorzugt 177 bis 185 kg/m$^3$ Wasser
- 0 bis 600 kg/m$^3$, vorzugsweise 1 bis 100 kg/m$^3$, besonders bevorzugt 5 bis 40 kg/m$^3$ Zusatzstoffe, insbesondere Flugasche und/oder Kalksteinmehl, und
- 0 bis 1,5 Gew.-%, vorzugsweise 0,01 bis 1,0 Gew.-%, besonders bevorzugt 0,1 bis 0,6 Gew.-% vom Zementgewicht Zusatzmittel, insbesondere Fließmittel.

7. Verfahren gemäß einem der Ansprüche 1 bis 6, **dadurch gekennzeichnet, dass** Hüttensand und/oder Flugasche als Zusatzstoff vom Typ 2 verwendet wird.

8. Verfahren gemäß einem der Ansprüche 1 bis 7, **dadurch gekennzeichnet, dass** mineralischer Füllstoff und/oder Silikastaub als Zusatzstoff vom Typ 1 verwendet wird.

9. Verfahren gemäß einem der Ansprüche 1 bis 8, **dadurch gekennzeichnet, dass** die Dichte des einströmenden Restwassers mit einem optischen Sensor, der die Trübung des Restwassers bestimmt, gemessen wird.

Europäisches
Patentamt

European
Patent Office

Office européen
des brevets

# EUROPÄISCHER RECHERCHENBERICHT

**Nummer der Anmeldung**

EP 21 21 5599

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A,D | DE 197 47 283 A1 (ELBA WERK MASCHINEN GMBH & CO [DE]) 10. Juni 1999 (1999-06-10) <br> * Seite 2, Zeile 29 - Zeile 36 * <br> * Ansprüche 1-3 * <br> ----- | 1-9 | INV. <br> C04B28/02 <br> C04B40/00 <br> C04B40/06 <br> G01N33/38 |
| A | DE 42 44 616 A1 (BIKOTRONIC IND ELEKTRONIC GMBH [DE]) 7. Juli 1994 (1994-07-07) <br> * Spalte 3, Zeile 47 - Spalte 4, Zeile 44 * <br> * Ansprüche 1,5-7 * <br> ----- | 1-9 | |
| A,D | WO 2020/030729 A1 (HAHN, BERND) 13. Februar 2020 (2020-02-13) <br> * Ansprüche 1,38,39,43,44 * <br> ----- | 1 | |
| A | EP 3 357 581 A1 (HAHN BERND [DE]) 8. August 2018 (2018-08-08) <br> * Ansprüche 1,6,8,14 * <br> ----- | 1 | |
| A | DE 295 22 219 U1 (DYCKERHOFF AG [DE]) 17. August 2000 (2000-08-17) <br> * Absatz [0014] * <br> * Anspruch 1 * <br> ----- | 1 | RECHERCHIERTE SACHGEBIETE (IPC) <br><br> C04B <br> G01N |

Der vorliegende Recherchenbericht wurde für alle Patentansprüche erstellt

| Recherchenort | Abschlußdatum der Recherche | Prüfer |
|---|---|---|
| Den Haag | 7. Juni 2022 | Kolb, Ulrike |

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 21 21 5599

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

07−06−2022

| Im Recherchenbericht angeführtes Patentdokument | Datum der Veröffentlichung | Mitglied(er) der Patentfamilie | Datum der Veröffentlichung |
|---|---|---|---|
| DE 19747283 A1 | 10−06−1999 | KEINE | |
| DE 4244616 A1 | 07−07−1994 | KEINE | |
| WO 2020030729 A1 | 13−02−2020 | DE 202019005624 U1<br>WO 2020030729 A1 | 23−03−2021<br>13−02−2020 |
| EP 3357581 A1 | 08−08−2018 | DE 102017102351 A1<br>EP 3357581 A1 | 09−08−2018<br>08−08−2018 |
| DE 29522219 U1 | 17−08−2000 | DE 29522219 U1<br>ES 2108675 T1<br>GR 3031729 T3 | 17−08−2000<br>01−01−1998<br>29−02−2000 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 19747283 A1
- DE 202019005624 U1
- WO 2020030729 A1